# EUROPEAN PATENT APPLICATION

(11) **EP 1 081 131 A1**
(43) Date of publication of application: **07.03.2001**
(21) Application number: 99922691.3
(22) Date of filing: 18.05.1999
(51) Int. Cl.: C07C 251/86, C07D 213/88, C07C 47/575, A61K 31/351, A61K 31/15, A61K 31/11, A61P 31/06

(54) **SELECTIVE ANTI-MYCOBACTERIAL PREPARATIONS, METHOD FOR PRODUCING THE SAME AND PHARMACEUTICAL COMPOSITIONS**

(30) Priority: 19.05.1998 RU 98109494
(71) Applicant: Institut Organicheskogo Sinteza Uralskogo Otdeleniya Rossiiskoi Akademii Nauck, Ekaterinburg 620219 (RU)
(72) Inventor: CHUPAKHIN, Oleg Nikolaevich, Ekaterinburg, 620075 (RU); FEDOROVA, Olga Vasilievna, Ekaterinburg, 620100 (RU); RUSINOV, Gennady Leonidovich, Ekaterinburg, 620142 (RU); MORDOVSKOI, Georgy Georgievich, Ekaterinburg, 620075 (RU); KHOMENKO, Alexandr Grigorievich, Moscow, 107014 (RU); GOLYSHEVSKAYA, Valentina Ivanovna, Moscow, 123308 (RU); ZUEVA, Marina Nikolaevna, Ekaterinburg, 620042 (RU); OVCHINNIKOVA, Irina Georgievna, Ekaterinburg, 620012 (RU)
(74) Representative: HOFFMANN - EITLE
(86) International application number: RU9900165
(87) International publication number: WO9959961

(57) **Abstract**

The present invention relates to new non-cyclic analogues of crown ethers (podands). principally di(formylaryl)polyethers, as well as to their hydrazones comprising substituted hydrazines of formula (I) where Ar is substituted phenylen or naphtylen. Y is an atom of hydrogen, halogen or a metoxy group, R is an oxygen atom or a NNH-R₁ group, R₁ is substituted phenyl, benzoyl or a 2- or 4-nicotinoyl. n is an integer between 1 and 3 and m is an integer between 1 and 4. This invention also relates to pharmaceutically acceptable addition salts and co-ordination compounds used as selective anti-mycobacterial preparations, as well as to a method for producing the same and to pharmaceutical compositions. The compounds of the present invention can essentially be used as an active basis in pharmaceutical preparations used for treating patients affected by mycobactenoses, including tuberculosis. These compounds consist of anti-mycobacterial preparations having a high specificity towards mycobacteria, a high activity against atypical, typical and resistant attains of mycobacteria, as well as a low toxicity. These compounds can be used in practical medicine for treating patients infected by sensitive and resistant as well as by typical and atypical strains of mycobacteria.

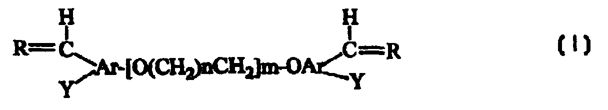

## Description

### TECHICAL FILD

The present invention relates to new noncyclic analogs of crown esters (podands), concretely to di(formyl aryl)polyesters, their hydrazones with substituted hydrazines, and also to pharmaceutically acceptable additive salts and coordination compounds and to methods of preparing them. The compounds being patented may be used, in the first place, as an active principle of pharmaceutical preparations for treatment of patients ill with mycobacteriosis, including tuberculosis.

### BACKGROUND ART

Widely known are industrially produced oxyethylene surfactants of the Triton-20 type, which have average antituberculosis activity in tests in vivo in the absence of activity in tests in vitro (Nature, 1987, 29[10], 2219).

One of the widely used agents for treatment of tuberculosis is phthivazid, formula I [M.D. Mashkovsky, Medicinal Agents, Kharkov, Torgsin, 1998, v. 2, p. 334], prepared by heating initial reagents as which hydrazide of isonicotinic acid and vanilline are used in the presence of alcohol, water and acid. The product being obtained is separated by filtering, washing and dehydrating.

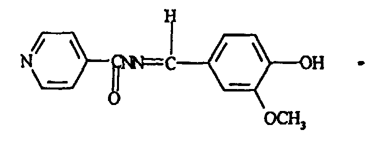

It is known the use of hydrazones of hydrazides of isonicotinic, nicotinic and benzoyl acids with substituted aromatic aldehydes to increase specificity to mycobacteria. Thus, for example, known is 1-(4-amino benzoyl)-2-benzal hydrazine, which is prepared by holding an alcohol solution of the initial reagents at 25°C for 24 hours (J. Ind. Chem.. Soc., 1984, 61[8], pp. 718-720). The yield of the desired product is 87%.

The analogs most similar to the claimed anti-mycobacterial preparations are 2-(allyl thio)-6-(2-chlorobenzoyl amino)benzothiazole of formula II (Czechoslovakian patent No. 239438, IPC C 07 D 277/64, 1987) and hydrazide of isonicotinic acid (isoniazid) of formula III, which is an active principle of a pharmaceutical composition serving for treatment of patients with mycobacteriosis [M.D. Mashkovsky, Medicinal Agents, v. 2, Kharkov, Torgsin, 1998, p. 332].

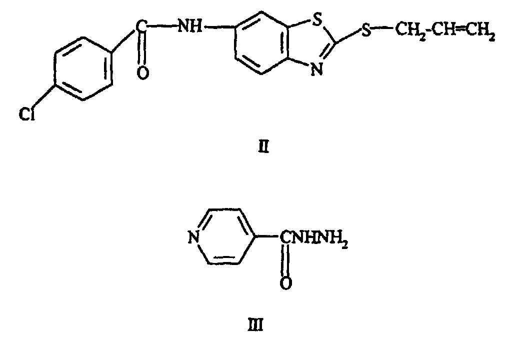

Increased toxicity and different side effects when they are used are known to person skilled in the art. [M.D. Mashkovsky, Medicinal Agents, v. 2, Kharkov, Torgsin, 1998, p. 332, and Pharmacology and Toxicology, 1987, 50, (4), p. 87]. The wide spread of clinical strains of mycobacteria resistant to isoniazid has been noted.

### DISCLOSURE OF THE INVENTION

The object of the invention is to synthesize antimycobacterial preparations with enhanced specificity while simultaneously reducing side effects with use and expanding the spectrum of antimycobacterial activity by providing the action thereof on typical and atypical, sensitive and resistant strains of mycobacteria.

This object is achieved in that substituted di(formyl aryl)polyesters of the general formula (IV), and also their pharmaceutical acceptable additive salts of general formula (V) and coordination compounds of general formula (VI) are used as antimycobacterial preparations. 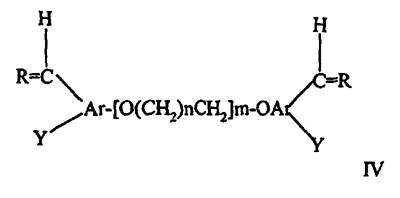 wherein Ar is a substituted phenylene or naphthalene; Y is a hydrogen atom, halogen or methoxy group; R is an oxygen atom, NNH-R₁-group; R₁ is a substituted phenyl, benzoyl, 2- or 4-nicotinoyl, n is an integer of from 1 to 3; m is an integer from 1 to 4; 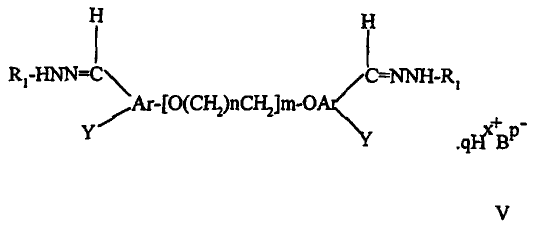 wherein Ar is a substituted phenylene or naphthalene; R₁ is a substituted phenyl, benzoyl, 2- or 4-nicotinoyl; Y is a hydrogen atom, halogen or methoxy group, n is an integer of from 1 to 3; m is an integer from 1 to 4; B is an anion of a mineral, alkyl- or aryl carboxylic acid, x, q and p are integers from 1 to 3. 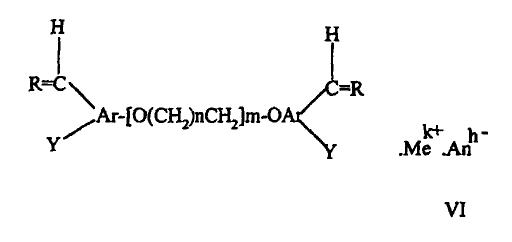 wherein Ar is a substituted phenylene or naphthalene; R is an oxygen atom or N-NH-R₁-group; R₁ is a substituted phenyl, benzoyl, 2- or 4-nicotinoyl, Y is a hydrogen atom, halogen or methoxy group; Me is a cation of mono-. bi-, tri- or tetravalent metal, An is an anion of mineral acid, n and h are integers of from 1 to 3, m is an integer of from 1 to 4, k is 1, 2, 3 or 4.

Cations of Li, Na, K, Cu; Co, Ni; Fe or Zr may be used as cations of metals Me.

A method of preparing a group of compounds of formula (IV) consists of obtaining di(formyl aryl)polyester of the general formula 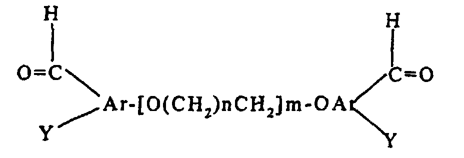 by the reaction of alkylating substituted aromatic aldehyde with dichloro- or dithiosyl-substituted glycols while heating and stirring reagents in dimethylformamide of formula C₃H₇NO (DMFA) in the presence of alkali in a medium of inert gas. The obtained di(formyl aryl)polyester is dissolved in an organic solvent and a general condensation reaction is carried out with an aqueous solution of substituted hydrazine of general formula R₁NHNH₂ (VII) wherein R₁is a substituted phenyl, benzoyl, 2- or 4-nicotinoyl.

The reaction is carried out while heating with the use of a promoting action, obtaining compounds of the formula (IV). The promoting action is provided by ultrasound or a weak organic or inorganic acid, for example, acetic (CH₃COOH), carbonic (H₂CO₃) and others. DMFA or alcohol of the general formula R₂-OH (VIII) in which R₂ is C₁-C₆ alkyl is used as the organic solvent. The desired product is separated by filtration and/or is purified, for example, by washing, crystallization and other known methods.

Compounds of formula (V) are prepared by a reaction of condensing di(formyl aryl)polyester, preliminarily dissolved in an organic solvent, and an aqueous solution of substituted hydrazine of formula (VII), using promoting action while heating to obtain a compound of formula (V). The promoting action is carried out with ultrasound and/or a reaction is carried out in the presence of a strong organic or inorganic acid. DMFA or alcohol (VIII) is used as the organic solvent. Hydrochloric (HCl), sulfuric (H₂SO₄), phosphoric (H₃PO₄), citric (C₆H₈O₇), tartaric (C₄H₆O₆) and other known acids are used as the strong acid. The product of interest is separated by filtration and/or purified, for example, by washing, crystallization and other known methods.

Compounds of formula (VI) are prepared by a reaction of condensing di(formyl alyl)polyester, preliminarily dissolved in an organic solvent, and an aqueous solution of substituted hydrazine of formula (VII), using promoting action while heating to obtain a compound of formula (VI). The promoting action is carried out with ultrasound or a reaction is carried out in a medium of a weak organic or inorganic acid, for example acetic. The obtained hydrazones (IV) in the form of a base are separated by filtration, and/or purified, for example by washing, crystallization and other known methods. Coordination compounds are obtained by mixing equimolar amounts of the compound (IV) and a metal salt in the medium of the organic solvent while heating or with sonochemical action. DMFA or alcohol (VIII) is used as the organic solvent.

A pharmaceutical composition is prepared by mixing a pharmaceutically acceptable filler and an effective amount of compounds of formula IV, V or VI. Any solid, liquid or plastic substance known for these purposes may be used as the filler.

The synthesis and use of substituted di(formyl aryl)polyesters, their pharmaceutical acceptable additive salts and coordination compounds as antimycobacterial preparations are related to each other by a single inventive concept.

As distinctive over known analogs in respect to pharmaceutical action, the claimed compounds are a new class of compounds and their derivatives, which are used for treatment of mycobacteriosis. The antimycobacterial activity of the claimed compounds as compared with the most similar analogs is presented in Table 1. The increased activity of the claimed compounds may be due to the fact that they comprise a polyester fragment of a certain length, which gives them the capability of carrying neutral molecules and charged particles, in particular metal cations, through lipophilia membranes (Table 2), and, apparently, ensures enhanced permeability of the claimed compounds through cellular membranes of the excitant.

These specific features of the structure and behavior result in an expansion of the spectrum of antimycobacterial activity: action on resistant mycobacteria (Tables 3, 4), action on typical and atypical mycobacteria (Table 5), and also increased specificity of the claimed compounds (Tables 6, 7). Due to its specificity to mycobacteria, the claimed compounds do not have a suppressing activity on normal microflora of the organism, are virtually nontoxic (Table 8), have a high therapeutic effect during treatment of experimental animals (Table 9).

### BEST METHOD OF CARRYING OUT THE INVENTION

### Di(formyl aryl)polyesters of the formula

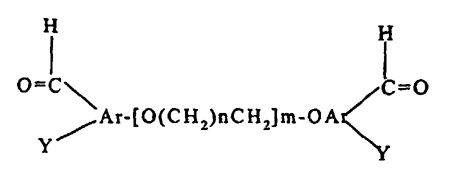

are prepared by dissolving a substituted aromatic aldehyde and alkali in DMFA at 60-70°C, adding in one half of an hour a solution of dichloro- or dithiozyl substituted glycol into DMFA, and subsequently heating the obtained mixture at 110-120°C for 15 hours in an inert gas atmosphere. The product of interest is separated by filtering, is purified by crystallization or chromatography on a column filled with aluminum oxide.

Hydrazones of di(formyl aryl)polyesters are prepared by mixing at 40-45°C solutions of di(formyl aryl)polyster in DMFA and substituted hydrazine of formula VII in a diluted, weak, for example, acetic acid, mixing for 5 minutes, holding at room temperature and subsequently filtering, washing and crystallizing.

Hydrazones of di(formyl aryl)polyesters are also prepared by ultrasound processing of a suspension of di(formyl aryl)polyester and substituted hydrazine of formula VII in an alcohol solution for 3-5 minutes with subsequent filtration and washing of the product of interest with alcohol.

Compounds of formula V are prepared by mixing, at 45-50°C, solutions of di(formyl aryl)polyester in DMFA and hydrazine of formula VII in a diluted aqueous solution of a strong inorganic or organic acid, for example, hydrochloric, mixing the obtained reaction mass for 25 minutes, holding at room temperature for 1 hour with subsequent filtration and reprecipitation from the alcohol with diethyl ether.

Compounds of formula V are also prepared by ultrasound processing for 3 minutes of a suspension of equimolar amounts of a hydrazone of di(formyl aryl)polyester and an according acid in alcohol with subsequent filtration and washing the product of interest with alcohol.

Compounds of formula VI are prepared by heating equimolar amounts of hydrazone and a metal salt in DMFA at 140-145°C for 10 minutes, the solvent is removed in a vacuum, with subsequent washing and crystallizing the product of interest.

Compounds of formula VI are also prepared by ultrasound processing for 10 minutes of equimolar amounts of hydrazone and a salt of mono-, bi-, tri- or tetravalent metal in alcohol and subsequently filtering and washing the product of interest with alcohol.

Using suspensions, not solutions, of the initial reagents, makes it possible to reduce the consumption of the solvents, to replace toxic or expensive solvents. Furthermore, the use of ultrasound action is characterized by a substantial increase in the selectivity of the process, which is expressed in an increase in the yield of the product of interest, right up to quantitative output.

Purification of the prepared compounds is controlled by the TLC method. The meanings of R_{f} of the claimed compounds were determined using "Sorbfil" plates (Silicagel CTX-1A, UV) (Russia) or "Silufol- UV" plates (Czechoslovakia). The composition and structure of the new compounds were confirmed by element analysis (C,H,N,O analyzer of the Karlo Erba firm) and also IR (Specord-UV-.751R) and PMR spectroscopy (Tesla BS-597A, 100 MHz). An UZDN-2T ultrasound disperser at a frequency of 22 kHz was used to carry out the reaction with ultrasound action. The cation-carrying properties of the claimed compounds were studied in accordance with a generally accepted method, which consists of studying the carrying of a cation from one aqueous phase to another aqueous phase through a layer of a nonpolar organic solvent.

### Example 1.

### 1,2-Bis(2-formyl-5-chlorphenoxy)-ethane

Added to a solution of 19.6 g (0.14 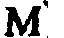 ) of 5-chlorosalicyl aldehyde in 200 ml of DMFA were 5.6 (0.14 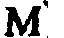 ) of NaOH and heated at a temperature of 60-70°C in an argon current to complete solution of the alkali. Then 6.93 g (0.07 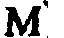 ) of dichloroethane in 50 ml of DMFA were added during half an hour. After that the temperature was raised to 110-120°C and mixing continued at that temperature for 15 hours. After cooling, the reaction mass was filtered and precipitated in 500 ml of water. The precipitated residue was filtered, washed with water. Purified by crystallization from the mixture DMFA:ethanol:water with carbon and aluminum oxide. Dried at a temperature of 100-120°C. R_{f}=0.75 (ethanol), melting point 82-84°C.
Yield=75%
Calculated for C₁₆H₁₂O₄Cl₂ MW 339
   C=56.64 H=3.54 Cl=20.94
Found
   C=56.45 H=3.43 Cl=20.53
IR spectrum, ν cm⁻¹: 1685 (C=O), 1250, 1040(C-O-C).
PMR spectrum, δ m.d.: 10.22(c,2H,H-C-O); 7.78-7.34( 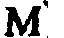 ,6H,C₆H₄),4.59(c,4H,OCH₂).

### Example 2

### 1,9-Bis(2-formyl phenoxy)-5-oxanonan

Prepared similar to example 1 from 12.2 g (0.1 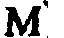 ) of salicyl aldehyde and 1.00 g (0.05 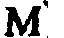 ) of dichlorodibutyl ester. Purified by recrystallization from methanol. R_{f}=0.78 (ethanol), melting point 197°C, Yield=77%
Calculated for C₂₂H₂₆O₅ MW 370
   C=71.35 H=7.03
Found:
   C=71.28 H=7.10
IR spectrum, ν, cm⁻¹: 1680 (C=O), 1255, 1145, 1080, 1040 (C-O-C).
PMR spectrum, δ m.d.: 12.08(c,2H,H-C-O); 8.21-6.94( 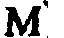 ,8H, aromatic protons); 4.13-3.45 (m, 8H,OCH₂); 1.76(c,8H,CH₂).

### Example 3

### 1,5-Bis(2-isonicotinoyl hydrazonomethyl phenoxy)-3-oxapentane

Added to a solution of 0.94 g (0.003 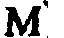 ) of 1,5-bis(2-formyl phenoxy)-3-oxapentane in 30 ml of DMFA were a solution of 0,.83 g (0.006 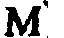 ) of a hydrazide of isonicotinic acid in 30 ml of 20% CH₃COOH at 45-50°C and energetically mixed at that temperature for 5 minutes, the precipitating residue was filtered, washed with water. Purified by crystallization from the mixture DMFA:ethanol. R_{f}=0.29 (acetones), melting point 230-232°C, Yield=79%
Calculated for C₃₀H₂₈N₆O₅ MW 552
   C=65.22 H=5.07 N=15.30
Found:
   C=65.28 H=4.92 N=15.59
IR spectrum, ν, cm⁻¹: 3200 (NH), 1679 (C=N), 1655 (C=O), 1555 (NH), 1245, 1150, 1115, 1080 (C-O-C).
PMR spectrum, δ m.d.: 12.09 (c,2H,HN-C-O); 8.81 (c,2H,H-C=N); 8.75-7.04 ( 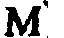 ,16H,aromatic protons); 3.72-4.30 ( 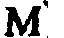 ,8H,OCH₂).

### Example 4

### 1,5-Bis(2-isonicotinoyl hydrazonomethyl phenoxy)-3-oxapentane

A suspension of 0.94 g (0.003 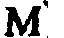 ) of 1.5-bis(formyl phenoxy)-3-oxapentane and 0.83 g (0.006 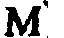 ) of a hydrazide of isonicotinic acid in 20 ml of 80% ethanol was subjected to the action of ultrasound with the use of the UZDN-2T device (frequency -22 kHz) for 3-5 minutes (checking with TLC), then the residue was filtered and purified by washing with ethanol, drying in vacuum. R_{f}=0.29 (acetone), melting point 230-232°C, Yield=95%
Calculated for C₃₀H₂₈N₆O₅ MW 552
   C=65.22 H=5.07 N=15.30
Found:
   C=65.35 H=5.22 N=15.47
IR spectrum, ν, cm⁻¹: 3200 (NH), 1679 (C=N), 1655 (C=O), 1555 (NH), 1245, 1150, 1115, 1080 (C-O-C).
PMR spectrum, δ m.d.: 12.09 (c,2H,HN-C-O); 8.81 (c,2H,H-C-N); 8.75-7.04 ( 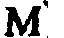 ,16H,aromatic protons); 3.72-4.30 ( 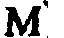 ,8H,OCH₂).

### Example 5

### 1,8-Bis[2-nicotinoyl hydrazonomethyl naphthoxy-2)]-3,6-dioxaoctane

Prepared in a manner similar to example 3 from 1.37 g (0.003 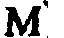 ) of 1,8-bis[2(formyl naphthoxy-2)]-3,6-dioxaoctane and 0.83 g (0.006 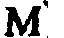 ) of a hydrazide of nicotinic acid, R_{f}=0.03 (acetone), melting point 82-84°C, Yield=78%
Calculated for C₄₀H₃₆N₆O₆ MW 696
   C=68.97 H=5.17 N=12.07
Found:
   C=68.70 H=5.25 N=12.00
K spectrum, ν, cm⁻¹: 3245 (NH), 1683 (C=N), 1652 (C=O), 1560 (NH), 1242, 1140, 1080, 1059 (C-O-C).
PMR spectrum, δ m.d.: 12.08 (c,2H,HN-C=O); 9.14 (c,2H,H-C=N); 7.59-7.20 ( 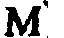 ,20H,aromatic protons); 4.33-3.85 ( 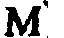 ,12H,OCH₂).

### Example 6

### 1,2-Bis(2-methoxy-4-benzoyl hydrazonomethyl phenoxy)-ethane

Prepared in a manner similar to example 4 from 0.99 g (0.003 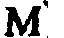 ) of 1,2-bis(2-methoxy-4-formyl phenoxy)-ethane and 0.82 g (0.006 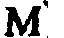 ) of a hydrazide of benzoic acid in 20 ml of 50% methanol. R_{f}=0.74 (acetone), melting point 258-260°C, Yield=97%.
Calculated for C₃₂H₃₀N₄O₆ MW 566
   C=68.84 H=5.30 N=9.89
Found:
   C=67.65 H=5.66 N=10.25
IR spectrum, ν, cm⁻¹: 3240 (NH), 1690 (C=N), 1650 (C=O), 1560 (NH), 1285, 1080(C-O-C).
PMR spectrum, δ m.d.: 11.75 (c,2H,HN-C=O); 8.39 (c,2H,H-C=N); 7.90-7.17 ( 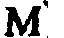 ,16H,aromatic protons); 4.39 (c,4H,OCH₂); 3.83 (c,6H,OCH₃).

### Example 7

### 1,2-Bis(5-chlor-2-isonicotinoyl hydrazonomethyl phenoxy)ethane

Prepared in a manner similar to example 4 from 1.02 g (0.003 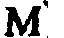 ) of 1,2-bis(5-chlor-2-formyl phenoxy)-ethane and 0.83 g (0.006 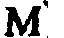 ) of a hydrazide of isonicotinic acid in 20 ml of isopropanol. R_{f}=0.43 (acetone), melting point 65-267°C. Yield=94%
Calculated for C₂₈H₂₂N₆O₄Cl₂ MW 577
   C=58.23 H=3.81 N=14.56
Found:C=58.99 H=3.58 N=14.44
IR spectrum, ν, cm^{-¹}: 3260 (NH), 1670 (C=N), 1650 (C=O), 1555 (NH), 1265, 1065 (C-O-C).
PMR spectrum, δ m.d.: 12.13 (c,2H,HN-C=O); 8.76 (c,2H,H-C=N); 8.71-7.02 ( 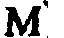 ,14H,aromatic protons); 4.49 (c,4H,OCH₂).

### Example 8

### 1.11-Bis(2-isonicotinoyl hydrazonomethyl phenoxy)-3,6,9-trioxaundecane

Prepared in a manner similar to example 4 from 1.21 g (0.003 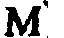 ) of 1.11-bis(2-formyl phenoxy)-3,6,9-trioxaundecane and 0.83 g (0.006 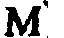 ) of a hydrazide of isonicotinic acid. R_{f}=0.17 (acetone), melting point 68-70°C, Yield=98%
Calculated for C₃₄H₄₀N₆O₈ MW 658
   C=62.01 H=5.78 N=12.77
Found:
   C=62.12 H=5.66 N=12.58
IR spectrum, ν, cm⁻¹: 3220 (NH), 1680 (C=N), 1658 (C=O), 1560 (NH), 1240, 1150, 1115, 1068 (C-O-C).
PMR spectrum, δ m.d.: 12.13 (c,2H,HN-C=O); 8.81 (c,2H,H-C=N); 7.00-7.83 ( 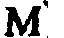 ,16H,aromatic protons); 4.22-3.56 ( 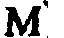 ,16H,OCH₂).

### Example 9

### 1,5-Bis(2-phenyl hydrazonomethyl phenoxy)-3-oxapentane

Prepared in a manner similar to example 3 from 2.88 g (0.02 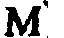 ) of a chlorohydrate of phenyl hydrazine and 2.86 g (0.01 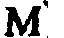 ) of 1,5-bis(2-formyl phenoxy)-3-oxapentane. R_{f}=0.33 (acetone), melting point 169-170°C, Yield 84%
Calculated for C₃₀H₂₈N₄O₃ MW 494
   C=72.89 H=6.07 N=11.34
Found:
   C=72.46 H=5.97 N=11.09
IR spectrum, ν, cm⁻¹: 3330 (NH), 1685 (C=N), 1565 (NH), 1256, 1160, 1116, 1050(C-O-C).
PMR spectrum, δ m.d.: 10.36 (c,2H,NH); 8.21 (c,2H,6H-C=N); 6.72-7.81 ( 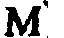 ,18H,aromatic protons); 3.96-4.24 ( 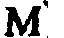 ,8H,OCH₂).

### Example 10

### 1,5-Bis(2-methoxy-4-nicotinoyl hydrazonomethyl phenoxy)-3-oxapentane trisulfate

Solutions of 0.94 g (0.003 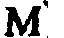 ) of 1,5-bis(2-formyl phenoxy)-3-oxapentane in 30 ml of DMFA and 0.83 g (0.006 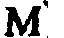 ) of a hydrazide of nicotinic acid in 30 ml of 20% sulfuric acid were joined at 45-50°C, mixed for 20 minutes, and then held at room temperature for 1 hour, filtered. The product was purified by precipitating from methanol with ether. Dried in vacuum, melting point 185-186°C, Yield 72%
Calculated for C₃₀H₂₈N₆O₅+3H₂SO₄ MW 748
   C=48.13 H=4.28 N=11.23 S=8.56
Found
   C=48.08 H=4.64 N=11.36 S=9.09
IR spectrum, ν cm⁻¹: 2700-2650, 2100, 1670 (C=N⁺H); 1250, 1165, 1050, 1030 (C-O-C).

### Example 11

### 1,8-Bis[2-(nicotinoyl hydrazonomethyl napbthoxy-2)]-3,6-dioxaoctane citrate

A suspension of 0.14 g (0.0002 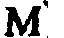 ) of 1,8-bis[2-(nicotinoyl hydrazonomethyl naphthoxy-2)]-3,6-dioxaoctane and 0.077 g (0.0004 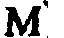 ) of citric acid was subjected to the action of ultrasound for 3 minutes, then cooled and filtered. The product was purified by washing with ethanol. Dried in vacuum, melting point 145-146°C, Yield 92%
Calculated for C₄₆H₄₆N₆O₁₄ MW 906
   C=60.93 H=5.07 N=9.27
Found
   C=60.83 H=4.71 N=9.20
IR spectrum, ν, cm⁻¹: 2650-2500, 1950, 1665 (C=N⁺H); 1249, 1111, 1083, 1043 (C-O-C), 1580 (COO⁻).

### Example 12

### [1,5-Bis(2-methoxy-4-formyl phenoxy)-3,6-dioxaoctane]potassium (1) isothiocyanate

A solution of 0.074 g (0.0002 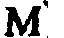 ) of 1,2-bis(2-methoxy-4-formyl phenoxy)-ethane and 0.02 g (0.0002 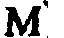 ) of KSCN in 20 ml of DMFA were boiled for 10 minutes, the solvent was distilled off in vacuum, the residue purified by recrystallization from ethanol. Dried in vacuum, melting point 135°C, Yield=68%
Calculated for C₂₁H₂₂NSK MW 471
   C=53.48 H=4.67 N=2.97
Found
   C=53.58 H=4.78 N=3.01
IR spectrum, ν, cm⁻¹: 1690 (C=O), 1265, 1150, 1070, 1050 (C-O-C).

### Example 13

### [1,8-Bis(3-nicotinoyl hydrazonomethyl phenoxy)-3,6-dioxaoctanej nickel(II)

Placed in 15 ml of methanol were 0.6 g (0.005 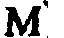 ) of 1,8-Bis(3-nicotinoyl hydrazonomethyl phenoxy)-3,6-dioxaoctane and 0.36 g (0.01 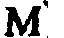 ) of Ni(NO₃)₂, a radiator of an ultrasound disperser was put in the flask and the device was turned on for 10 minutes. After cooling a residue precipitated, which was filtered, washed with hot methanol and dried in a vacuum, melting point above 250°C, Yield=90%
Calculated for C₃₂H₃₂N₆O₆Ni MW 655
   C=58.63 H=4.89 N=12.82
Found
   C=58.77 H=5.30 N=12.36
IR spectrum, ν, cm⁻¹: 3450, 3180 (NH), 1680 (C=N), 1650 (C=O), 1265, 1140, 1100, 1050 (C-O-C).

### Example 14

### [1,5-Bis(2-methoxy-4-nicotinoyl hydrazonomethyl phenoxy)-3-oxapentane] iron(III)

Prepared in a manner similar to example 13 from 0.5 g (0.0008 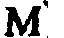 ) of 1,5-bis(2-methoxy-4-nicotinoyl hydrazonomethyl phenoxy)-3-oxapentane and 0.26 g (0.0016 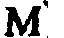 ) of FeCl₃, melting point above 250°C, Yield=91%
Calculated for C₃₄H₃₄N₄O₇Fe MW 665.8
   C=61.28 H=5.11 N=8.41
Found
   C=61.61 H=5.37 N=8.66
IR spectrum, ν, cm⁻¹: 3460, 3200 (NH), 1685 (C=N), 1655 (C=O), 1266, 1145, 1190, 1055 (C-O-C)

### Example 15

### Pharmaceutical composition on the base of 1,8-bis(2-isonicotinoyl hydrazonomethyl phenoxy)-3,6-dioxaoctane for investigation of therapeutic effect

21 mg of the preparation were ground in a mortar and 3 ml of a 1% starch solution were added in small portions until a homogeneous suspension was obtained. The prepared composition was used for treatment of Guinea pigs by administering 1 ml of the prepared suspension daily for several days. Data in Table 9.

### Example 16

### [1,8-Bis(3-isonicotinoyl hydrazonomethyl phenoxy)-3,6-dioxaoctanej copper (II) sulfate

2.98 g (0.005 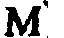 ) of [1,8-Bis(3-isonicotinoyl hydrazonomethyl phenoxy)-3,6-dioxaoctane] and 0.80 g (0.005 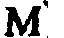 ) of CuCl2 were placed in 15 ml of methanol and a radiator of an ultrasound disperser was put in the flask and the device was turned on for 10 minutes. After cooling a residue precipitated, which was filtered, washed with hot methanol and dried in a vacuum, melting point above 250°C, Yield=97%
Calculated for C₃₂H₃₂N₆O₈SCu MW 915
   C=50.83 H=4.24 N=11.12
Found
   C=50.30 H=4.15 N=10.99
IR spectrum, ν, cm⁻¹: 1628 (C=N), 1600 (C=O), 1580 (NH), 1268, 1120, 1060, 1035 (C-O-C).

### Example 17

### [1,2-Bis(2-benzoyl hydrazonomethyl phenoxy)-ethane] cobalt (II)

2.53 g (0.005 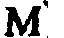 ) of 1,2-bis(2-benzoyl hydrazonomethyl phenoxy)-ethane and 0.89 g (0.005 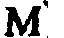 ) of Co(CH₃COOH)₂ were placed in 15 ml of methanol and a radiator of an ultrasound disperser was put in the flask and the device was turned on for 10 minutes. After cooling a residue precipitated, which was filtered, washed with hot methanol and dried in a vacuum, melting point above 250°C, Yield=89%
Calculated for C₃₂H₃₄N₄O₄Co MW 563
   C=63.94 H=4.24 N=9.95
Found
   C=63.75 H=4.31 N=10.33
IR spectrum, ν, cm⁻¹: 1625 (C=N), 1600 (C=O), 1585 (NH), 1265, 1025 (C-O-C).

### Example 18

### [1,5-Bis(2-isonicotinoyl hydrazonomethyl phenoxy)-3-oxapentane] zirconium (IV) disulfate

2.76 g (0.005 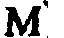 ) of 1,5-bis(2-isonicotinoyl hydrazonomethyl pbenoxy)-3-oxapentane and 1.78 g (0.005 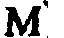 ) of Zr(SO₄)₂₌ 4H₂O were placed in 30 ml of DMFA and heated to complete dissolution of the components (5 minutes). The solvent was distilled off in a vacuum, the residue washed with ethanol and dried in a vacuum. Melting point above 250°C, Yield=95%
Calculated for C₃₀H₂₈N₆O₁₃S₂Zr MW 835.2
   C=43.10 H=3.35 N=10.06
Found
   C=43.40 H=3.05 N=10.25
IR spectrum, ν, cm⁻¹: 1630 (C=N), 1610 (C=O), 1590 (NH), 1260, 1120, 1080, 1040 (C-O-C).

A study of the antimycobacterial activity of the claimed compounds in experiments in vitro was carried out bacteriologically by the method of vertical diffusion on a dense Lievenstein-Yensen or "Novaya" nutrient. Each culture in an amount of 0.3 ml was implanted in a test tube with a test-microbe seeded along the free edge on the bottom. The test tube was placed in a thermostat in a vertical position and incubated at 37°C, the results were taken on the 10-12th day. The claimed preparations exhibited high antimycobacterial activity, especially in relation to atypical strains of mycobacteria (Table 5), and also to strains of mycobacteria which are resistant to the main antituberculosis preparations (Tables 3,4).

The antibacterial activity of the claimed compounds was studied with the method of seeding solutions of the preparation in a cavity. The test-microbe was put in a Petri dish containing a simple nutrient agar. The preparation in an amount of 0.02 ml in necessary concentrations was put in a cavity located in the middle of the Petri dish. The results were taken after 24-hour incubation in a thermostat at 37°C by determination of the zone of delay of growth of the microorganisms along the edges of the cavity.

A study of the therapeutic action of the claimed compounds was carried out on guinea pigs having an average weight of 350 g and infected subcutaneously in the region of the right groin with 0.001 mg/ml of the culture (strain H₃₇Rᵥ). Treatment began 2 weeks after infection and was carried out 5 times a week for 60 days. The preparation was given to guinea pigs in a dose of 21 mg/kg of the weight, a control group of pigs received isoniazid in an equivalent dose. The results are presented in Table 9. It is evident from the table that animals being studied, which received 1,8-bis(2-isonicotinoyl hydrazonomethyl phenoxy)-3,6-dioxaoctane, had a substantially different weight as compared with the control animals. There was a difference in the weight of the organs. In all of the control animals, tuberculosis damage was localized mainly at the point of administration. After the end of the course of treatment, tuberculosis changes were detected as morphological, not as bacteriological, in animals who had received isoniazid or the claimed preparation.

A study of acute toxicity was carried out according to a standard method on white mice. The animals received the preparation per os in a 0.5% solution of starch for 5 days. The values of LD₅₀ are presented in Table 8.

### INDUCTRIAL APPLICABILITY

The claimed compounds in respect to their characteristics (low toxicity, high specificity to mycobacteria, high activity in respect to atypical, typical, medicinal-resistant strains of mycobacteria, including strains of mycobacteria resistant to isoniazid) are superior to analogs in respect to both structure and action. The new compounds may be used in practical medicine for the treatment of patients, infected with sensitive and resistant, typical and atypical strains of mycobacteria.

**Table 1**

| Antimycobacterial activity of claimed preparations in tests In vitro (µg/ml) as compared with analogs as to effect | | | | | |
|---|---|---|---|---|---|
| Preparation | Strains of mycobacteria | | | | |
| | Typical | | Atypical | | |
| | *H*_{*37*}*R*_{*v*} | *M. bovis* | *M. smegmatis* | *M. avium* | *M.fortuirum* |
| Isoniazid formula (II) | 0.05 | 0.05 | 50.0 | 50.0 | 50.0 |
| Compound formula (II) | 10.0 | - | - | 25.0 | 10.0 |
| 1,5-Bis-(2-isonicotinoyl hydrazonomethyl phenoxy-3-oxapentane | 0.01 | 0.01 | 12.5 | 25.0 | 12.5 |
| 1,8-Bis-(2-isonicotinoyl hydrazonomethyl phenoxy)-3,6-dioxaoctane | 0.05 | 0.05 | 12.5 | 25.0 | 12.5 |
| 1,2-Bis-(2-formyl-5-chlorphenoxy) -ethane | 0.4 | 0.4 | 6.3-12.5 | 25.0 | 25.0 |

**Table 2**

| Speed of carrying cations through lipophil membrane 1,5-bis(2-phenyl hydrazonomethyl phenoxy)-3-oxapentane | |
|---|---|
| Cation of metal | Speed of carrying, mol h⁻¹ 10⁻⁷ |
| K | 34.5 |
| Na | 28.7 |
| Fe | 60.0 |
| Cu | 2.0 |

**Table 3**

| Bacteriostatic activity of claimed preparations in respect to resistant strains of mycobacteria of tuberculosis, taken from patients* | | |
|---|---|---|
| Compound | Inhibiting level, µg/ml | |
| | Strain resistant to isoniazid | Strain resistant to streptomycin and rifamycin |
| Isoniazid | 5.0 | 0.1 |
| Streptomycin | >50.0 | 10.0 |
| Rifamycin | - | 20.0 |
| 1,2-Bis[2(benzoyl hydrazonomethyl naphthoxy-2)]-ethane | 5.0 | 0.4 |
| 1,2-Bis(2-formyl-5-chlorophenoxy)-ethane | 5.0 | 0.4 |
| 1,2-Bis(2-methoxy-4-benzoyl hydrazonomethyl phenoxy)-ethane | >5.0 | 0.8 |
| 1,9-Bis(2-nicotinoyl hydrazonomethyl phenoxy)-5-oxanonon | >5.0 | 0.4 |

| | | |
|---|---|---|
| * Results obtained in Sverdlovsk Regional SRI of Phthisiopulmonology in 1998 | | |

**Table 4**

| Bacteriostatic activity of 1,5-bis(2-isonicotinoyl hydrazonomethyl phenoxy)--3-oxapentane in respect to resistant strains of mycobacteria of tuberculosis, taken from patients** | | |
|---|---|---|
| Characteristics of strains | Inhibiting | level, µg/ml |
| | Claimed preparation | Isoniazid of formula (III) |
| Sensitive to streptomycin, rifamycin and isoniazid | 0.58 | 0.29 |
| Resistant to streptomycin | 1.56 | 0.29 |
| Resistant to rifamycin | 0.20 | 0.29 |
| Resistant to isoniazid | 18.80 | >100 |
| Semiresistant (resistant to streptomycin, rifamycin and isoniazid | 1.57 | 2.125 |

| | | |
|---|---|---|
| ** Results obtained in Central SRI of Tuberculosis RAMS in 1997 | | |

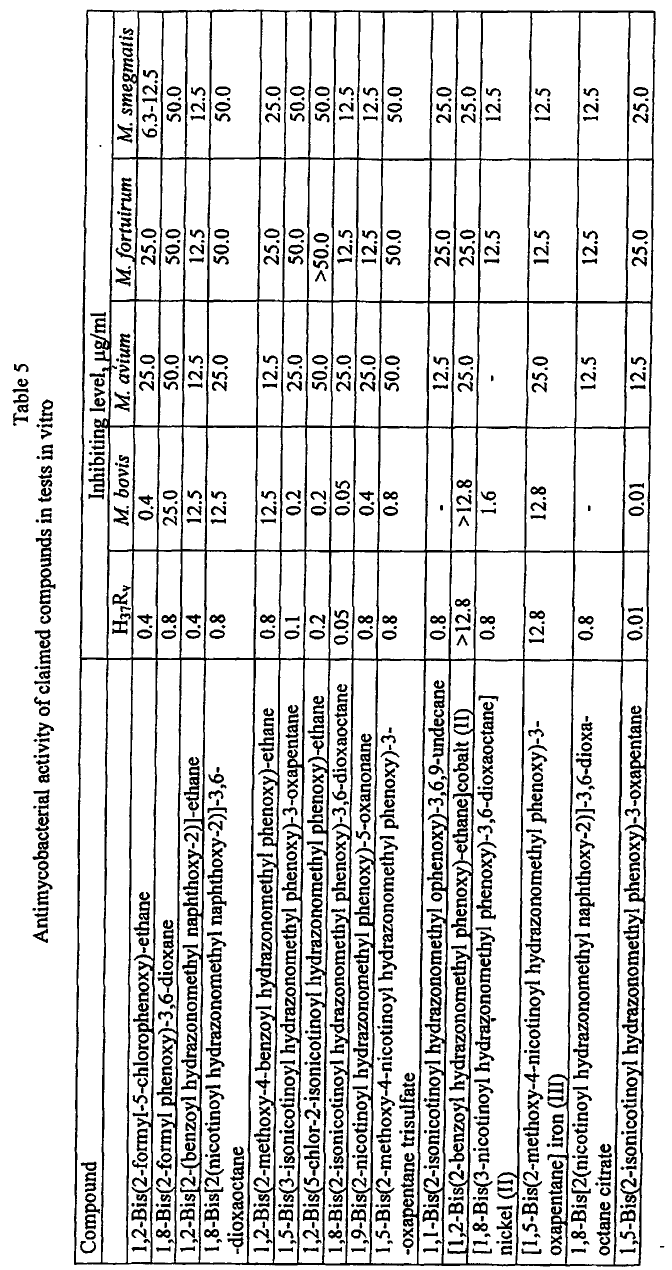

**Table 6**

| Antibacterial activity of claimed compounds in tests in vitro | | | | |
|---|---|---|---|---|
| | Inhibiting level, µg/ml | | | |
| Compound | *S. aureus* | *E. coli* | *P. vulgaris* | *Candida Albicans* |
| 1,5-Bis(2-isonicotinoyl hydrazono methyl phenoxy)-3-oxapentane | >500 | >500 | >500 | >500 |
| 1,8-Bis(2-isonicotinoyl hydrazono methyl phenoxy)-3,6-dioxaoctane | >500 | >500 | >500 | >500 |

**Table 7**

| Antibacterial activity of claimed compounds in tests in vitro | | | |
|---|---|---|---|
| Compound | Inhibiting level, µg/ml | | |
| | *S. epidermides* | *E. coli* | *Lactobacteria* |
| 1,8-Bis(2-formyl phenoxy)-ethane | >200 | >200 | >200 |
| 1,11-Bis(2-isonicotinoyl hydrazonomethyl phenoxy)-3,6,9-trioxaundecane | >50 | >50 | >50 |
| 1,2-Bis-(2-methoxy-4-benzoyl hydrazonomethyl phenoxy)-ethane | >50 | >50 | >50 |
| 1,2-Bis[2(benzoyl hydrazonomethyl naphthoxy--2)]-ethane | >50 | >50 | >50 |
| 1,8-Bis(3-nicotinoyl hydrazonomethyl phenoxy)--3,6-dioxaoctane] nickel (II) | >50 | >50 | >50 |
| 1,9-Bis(2-nicotinoyl hydrazonomethyl phenoxy)--5-oxanocan | >100 | >100 | >100 |

**Table 8**

| Study of acute toxicity of claimed compounds on mice (per os) | |
|---|---|
| Compound | LD₅₀, mg/kg |
| 1,2-Bis(2-formyl phenoxy)-3-oxapentane | >2000 |
| 1,5-Bis(2-benzoyl hydrazonomethyl phenoxy)-3-oxapentane | >1000 |
| 1,8-Bis(2-isonicotinoyl hydrazonomethyl phenoxy)-3,6-dioxaoctane | >2000 |
| Isoniazid | 363 |

**Table 9**

| Study of therapeutic action of 1,8-bis(2-isonicotinoyl hydrazonomethyl phenoxy)-3,6-dioxaoctane on guinea pigs (Strain H₃₇R₄) | | | |
|---|---|---|---|
| Group of pigs | Average change in weight of pigs during test, g | Weight of lungs, g | Weight of spleen, g |
| Control | 3.12±0.13 | 4.58±0.64 | 1.52±0.78 |
| Treatment with isoniazid (formula III) | 180.60±18.80 | 4.49±0.80 | 9.34±0.30 |
| Treatment with claimed preparation | 148.80±29.40 | 4.90±0.90 | 8.98±0.15 |

## Claims

1. Substituted di(formyl aryl)polyesters of the general formula A 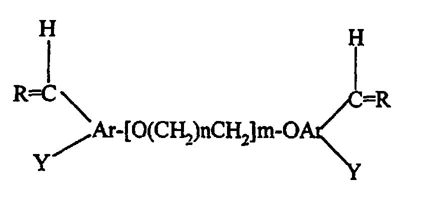 wherein
Ar is substituted phenylene or naphthylene;
Y is a hydrogen atom, halogen or methoxy group;
R is an oxygen atom, NNH-R₁-group;
R₁ is substituted phenyl, benzoyl, 2- or 4-nicotinoyl;
n is an integer of from 1 to 3;
m is an integer of from 1 to 4;
or their coordination compounds with cations of mono-, bi-, tri- or tetravalent metals or salts thereof, or their pharmaceutical acceptable additive salts of compounds A, in which R designates -NNH-R₁-group, with organic and inorganic acids, having antimycobacterial activity.

2. Pharmaceutically acceptable additive salts of compounds according to claim 1 of the general formula 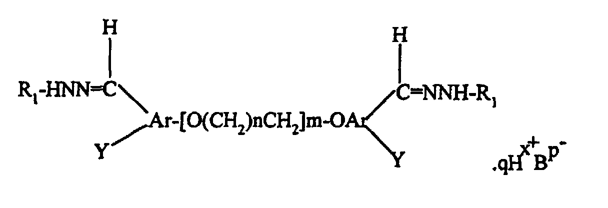 wherein
Ar is substituted phenylene or naphthylene;
R₁ is substituted phenyl, benzoyl or 2- or 4-nicotinoyl;
Y is a hydrogen atom, halogen or methoxy group;
n is an integer of from 1 to 3;
m is an integer of from 1 to 4;
B is an anion of mineral, alkyl- or arylcarboxylic acid;
x, q and p are integers of from 1 to 3.

3. Coordination compounds according to claim 1 of the general formula C 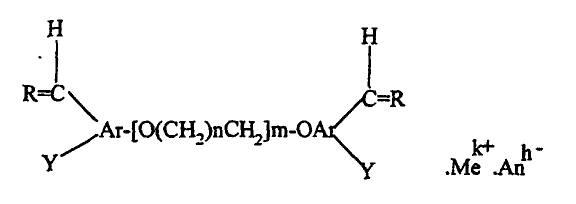 wherein
Ar is substituted phenylene or naphthylene;
R is an oxygen atom or -N-NH-R₁- group;
R₁ is substituted phenyl, beazoyl, 2- or 4-nicotinoyl;
Y is a hydrogen atom, halogen or methoxy group;
Me is a cation of mono-, bi, tri- or tetravalent metal;
An is an anion of mineral acid;
n and h are an integer of from 1 to 3;
m is an integer of from 1 to 4;
k is 1, 2, 3 or 4.

4. Compounds according to claim 3, which contain a cation of Li, Na, K, Cu, Co, Ni, Fe or Zr as a cation of the metal Me.

5. A method of preparing compounds according to claim 1, characterized in that a substituted aromatic aldehyde is subjected to alkylation with dichioro- or ditosyl-substituted glycols in the presence of an alkali in a medium of an organic solvent while heating, formed di(formyl aryl)polyester is subjected to a reaction of condensation with an aqueous solution of hydrazine of the general formula
R₁NHNH₂,
wherein R₁ is substituted phenyl, benzoyl or 2- or 4-nicotinoyl, in a medium of an organic solvent with the use of promoting action while beating, with subsequent separation of the product of interest or its conversion into a coordination compound or pharmaceutically acceptable additive salt.

6. A method according to claim 5, characterized in that dimethylformamide or alcohol is used as the organic solvent.

7. A method according to claim 5 and claim 6, characterized in that ultrasound or weak organic or inorganic acids are used as the promoting actions.

8. A method according to claim 5 and claim 6, characterized in that compounds of formula B according to claim 2 are prepared with the use of ultrasound as the promoting action and/or the reaction is carried out in the presence of a strong organic or inorganic acid.

9. A method according to claim 5 and claim 7, characterized in that compounds of formula C according to claim 3 are prepared by interaction of equimolar amounts of a compound of formula A and a salt of metal in a medium of an organic solvent while heating or with the action of ultrasound.

10. A pharmaceutical composition having antimycobacterial activity, containing an active principle and a pharmaceutically acceptable filler, characterized in that it comprises as the active principle substituted di(formyl aryl)polyesters according to claim 1 or their additive salts, or coordination compounds with cations of mono-, bi-, tri- or tetravalent metals or their salts in an effective amount.
